(19) Europäisches Patentamt
European Patent Office
Office européen des brevets

(11) EP 0 601 438 B1

(12) EUROPÄISCHE PATENTSCHRIFT

(45) Veröffentlichungstag und Bekanntmachung des Hinweises auf die Patenterteilung: **16.04.1997 Patentblatt 1997/16**

(51) Int. Cl.$^6$: **C07C 45/46**, C07C 205/45, C07C 49/784, C07C 49/80, C07C 49/813, C07C 49/84

(21) Anmeldenummer: **93119254.6**

(22) Anmeldetag: **30.11.1993**

(54) **Verfahren zur Herstellung mehrfach acylierter Aromaten**

Process for the preparation of polyacylated aromatics

Procédé pour la préparation de composés aromatiques polyacylés

(84) Benannte Vertragsstaaten:
**BE CH DE ES FR GB IT LI NL**

(30) Priorität: **05.12.1992 DE 4240966**

(43) Veröffentlichungstag der Anmeldung: **15.06.1994 Patentblatt 1994/24**

(73) Patentinhaber: **BASF Aktiengesellschaft**
**67063 Ludwigshafen (DE)**

(72) Erfinder:
- **Siegel, Wolfgang, Dr.**
**D-68167 Mannheim (DE)**
- **Schroeder, Jochen, Dr.**
**D-67117 Limburgerhof (DE)**

(56) Entgegenhaltungen:
**DE-C- 515 540**
**GB-A- 2 235 195**
**US-A- 4 922 026**

- **SYNTHESIS. 1972 , STUTTGART DE Seiten 533 - 542 D.E. PEARSON ET AL. 'Friedel - Crafts Acylations with little or no Catalyst'**
- **CHEMICAL ABSTRACTS, vol. 86, no. 25, 20. Juni 1977, Columbus, Ohio, US; abstract no. 189401w, YULDASHEV, KH. YU. ET AL. 'Chloroacetylation of aromatic hydrocarbons in the presence of small amounts of catalysts.' Seite 563 ;Spalte 2 ;**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist. (Art. 99(1) Europäisches Patentübereinkommen).

EP 0 601 438 B1

## Beschreibung

Die vorliegende Erfindung betrifft ein neues Verfahren zur Herstellung von mehrfach acylierten Aromaten der allgemeinen Formel I

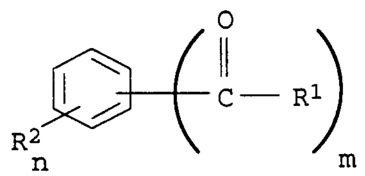
I

in der die Variablen folgende Bedeutung haben:

- $R^1$ Phenyl oder inerte Substituenten tragendes Phenyl, $\alpha$-Halogen-$C_1$-$C_6$-Alkyl, wobei diese Reste gleich oder verschieden sein können;
- $R^2$ $C_1$-$C_4$-Alkyl,
- m 2 oder 3;
- n 0 bis 4, wobei die Substituenten $R^2$ verschieden sein können, wenn $n > 1$ ist,

mit der Maßgabe, daß $m + n \leqslant 6$ ist.

Die Einführung einer zweiten Acylgruppe in Acylaromaten ist dadurch erschwert, daß Acylgruppen den Aromaten für weitere Friedel-Crafts-Acylierungen desaktivieren.

In Zh. Org. Khim. 6 (1970) 532 wird beschrieben, daß eine weitere Benzoylgruppe durch Reaktion mit Benzoylchlorid in Gegenwart katalytischer Mengen feinverteilten Eisenpulvers oder $FeCl_3$ in hochaktiviertes 2,4-Dimethoxybenzophenon eingeführt werden kann.

Eine direkte zweifache Friedel-Crafts-Acylierung von Trimethyl- und Tetramethylbenzolen in Gegenwart von $AlCl_3$ ist aus Org. Prep. Proc. Int. 10 (1978) 255 bekannt und für 1,2,4,5-Tetramethylbenzolderivate in der GB-A 22 35 195 offenbart. Dazu werden große molare Überschüsse an Carbonsäurechlorid und $AlCl_3$ benötigt, die nicht nur unwirtschaftlich sind, sondern auch bei der üblichen hydrolytischen Aufarbeitung zu salzsäurehaltigen Abwässern führen, die eine aufwendige Entsorgung nach sich ziehen.

Die direkte Umsetzung von Anisol, einem stark aktivierten Aromaten, mit Benzoylchlorid in Gegenwart von Eisenpulver zu 2,4-Dibenzoylanisol beschreibt Zh. Org. Khim. 6 (1970) 535.

Die einfache Acylierung von Aromaten mit katalytischen Mengen $FeCl_3$, Jod, $ZnCl_2$ und Eisenpulver ist aus Synthesis (1972) 533 bekannt.

Es bestand die Aufgabe, ein Verfahren zur Verfügung zu stellen, das es erlaubt, weitere Acylgruppen in unsubstituierte oder $C_1$-$C_4$-alkylsubstituierte Acylaromaten einzuführen, ohne daß dazu äquimolare Mengen an Friedel-Crafts-Katalysatoren benötigt werden. Weiterhin bestand die Aufgabe, ein Verfahren zu finden, das die unmittelbare Herstellung von mehrfach acylierten Aromaten mit nur katalytischen Mengen an Katalysatoren aus Aromaten in nur einem Verfahrensschritt erlaubt.

Diese Aufgabe wurde durch das oben definierte Verfahren gelöst, das dadurch gekennzeichnet ist, daß man Acylaromaten der allgemeinen Formel II

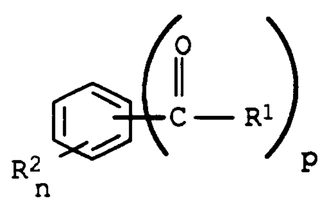
II

in der p für 1 oder 2 steht, mit einer dem gewünschten Acylierungsgrad entsprechenden Menge an einem Carbonsäurehalogenid der allgemeinen Formel III

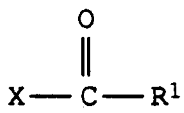

III

in der X für Halogen steht, in Gegenwart von Fe(II),-Fe(III)-, Zn(II)-, Mo(VI)-, W(VI)- oder Sn(IV)-Verbindungen umsetzt.

Schematisch läßt sich das Verfahren durch folgende Reaktionsgleichung wiedergeben:

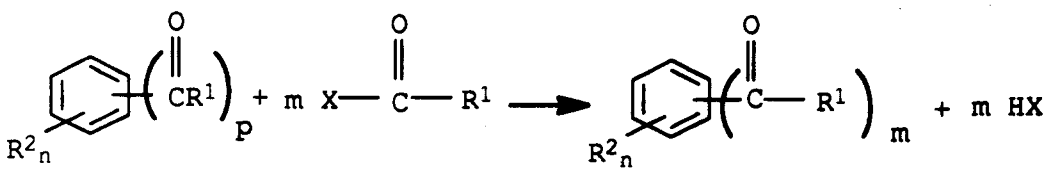

II III I

Die Ausgangsverbindungen II sind an sich bekannt oder nach bekannten Methoden erhältlich. Es handelt sich um entsprechende Phenyl- oder $C_1$-$C_4$-alkylsubstituierte Phenylverbindungen. Die Substituenten $R^2$ sind z. B. Ethyl, n-Propyl und n-Butyl, vorzugsweise aber Methyl.

In einer besonders bevorzugten Ausführungsform wird die Verbindung II in situ aus Aromaten IV hergestellt:

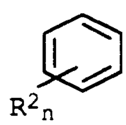

IV

Bei diesen Aromaten handelt es sich um Verbindungen wie Benzol, Toluol, o-Xylol, m-Xylol, p-Xylol und vor allem tri- und tetra-$C_1$-$C_4$-alkylsubstituierte Benzole wie 1,3,5-Trimethylbenzol (Mesitylen), 1,3,5-Triethylbenzol, 1,2,4,5-Tetramethylbenzol (Durol) und 1,2,3,5-Tetramethylbenzol (Isodurol).

Die Verbindungen II bzw. IV werden mit Carbonsäurehalogeniden III umgesetzt, von denen die Chloride bevorzugt sind. Es kommen zum einen Benzoylhalogenide oder solche Benzoylhalogenide in Betracht, die am Phenylrest unter den Reaktionsbedingungen inerte Substituenten wie Nitro-, Halogen wie Fluor, Chlor und Brom, $C_1$-$C_6$-Alkoxygruppen wie Methoxy und $C_1$-$C_4$-Alkylgruppen wie Methyl tragen. Weiterhin können aliphatische $C_1$-$C_6$-$\alpha$-Halogencarbonsäuren wie 2-Halogen-Essig- und 2-Halogen-Propionsäure im erfindungsgemäßen Verfahren eingesetzt werden. Als bevorzugte Beispiele seien genannt: Benzoylchlorid, 4-Fluorbenzoylchlorid, 4-Chlorbenzoylchlorid, 4-Nitrobenzoylchlorid, 2-Methylbenzoylchlorid und Chloracetylchlorid.

Die Reaktion wird durch eine Reihe definitionsgemäßer Metallverbindungen katalysiert. In Betracht kommen Fe(II)-Verbindungen wie $FeSO_4$, Fe(III)-Verbindungen wie $Fe_2O_3$, $FeCl_3$, $FeBr_3$, $Fe_2(SO_4)_3$, Eisencarboxylate wie Eisen(III)-Acetat, Eisen (III)-acetylacetonat, natürlich auch gemischt-valente Eisenverbindungen wie $Fe_3O_4$, weiterhin Zink(II)-Verbindungen wie ZnO, $ZnCl_2$, $ZnBr_2$, $ZnSO_4$, Zinkcarboxylate wie Zinkbenzoat und Zinkacetat, Molybdän- und Wolfram(VI)-Verbindungen wie $MoO_3$ und $WO_3$ sowie Zinn(IV)-Verbindungen wie Zinntetrachlorid oder -bromid. Die Eisenverbindungen sind als Katalysatoren bevorzugt, davon besonders Eisenoxid $Fe_2O_3$. Die genannten Verbindungen können in freier Form, aber auch an inerte Träger gebunden eingesetzt werden. Als Träger kommen z.B. Siliciumoxid, Aluminiumoxid oder Alumosilikate in Betracht.

Die molaren Verhältnisse der Verbindungen II und III können entsprechend dem gewünschten Acylierungsgrad variieren. Für eine Acylierung von bereits Acylgruppen tragenden Aromaten II liegen die Verhältnisse aber in der Regel bei 1:1 bis 1,3:1 Äquivalenten Carbonsäurehalogenid zum Aromaten. Für Zweifachacylierungen wird im allgemeinen ein Überschuß eingesetzt, so daß die molaren Mengen hier zwischen 3:1 und 10:1 für III:II liegen. Geht man direkt von Aromaten IV aus, muß entsprechend mindestens ein Äquivalent Carbonsäurehalogenid mehr eingesetzt werden.

Der Katalysator kann in Mengen von 0,01 bis 20 mol.-%, vorzugsweise 0,1 bis 3 mol-% bezogen auf die Menge des Acylaromaten II bzw. des Aromaten IV eingesetzt werden.

Die Reaktionstemperatur liegt in der Regel bei 40 bis 250°C, bevorzugt bei 80 bis 200°C und besonders bevorzugt bei 100 bis 180°C.

Der Druck spielt im erfindungsgemäßen Verfahren keine erkennbare Rolle. Die Reaktion kann deshalb bei 1 bis 10 bar ausgeführt werden, bevorzugt ist aber Normaldruck. Die Reaktion kann nach Vermischen der Ausgangsverbindungen und des Katalysators durch Erwärmen auf Reaktionstemperatur erfolgen. Bevorzugt wird aber, den Ausgangsstoff II bzw. IV und den Katalysator vorzulegen und bei erhöhter Temperatur mit dem Carbonsäurehalogenid III zu versetzen.

Die Reaktion kann in einer bevorzugten Ausführungsform ohne Lösungsmittel erfolgen, es können aber auch inerte Lösungsmittel wie Nitrobenzol oder Kohlenwasserstoffe mitverwendet werden.

Die Reaktion ist im allgemeinen nach 2 bis 6 Stunden beendet. Die Isolierung der Produkte erfolgt nach bekannten Methoden wie Destillation oder Kristallisation.

Das erfindungsgemäße Verfahren erlaubt die Herstellung mehrfach acylierter Aromaten aus einem Acylaromaten und Carbonsäurehalogeniden in guten Ausbeuten. Dazu werden nur katalytische Mengen einer Metallverbindung gebraucht. Die Reaktion kann ohne Lösungsmittel durchgeführt werden und erfordert keine hydrolytische Aufarbeitung. Weiterhin erlaubt die Erfindung die Herstellung mehrfach acylierte Aromaten aus Benzol oder $C_1$-$C_4$-alkylsubstituierten Benzolen.

Die verfahrensgemäß hergestellten Produkte finden als Monomerbausteine für Polyarylether (GB 2 235 195) Verwendung.

## Beispiele

### Allgemeine Vorschrift für die Beispiele

1 mol eines substituierten Benzols IV und Katalysator wurden bei der Temperatur $T_1$ mit dem Carbonsäurechlorid III versetzt und für eine Zeit t auf die Temperatur $T_2$ erhitzt. Die Isolierung erfolgte destillativ oder durch Kristallisation aus Heptan.

Die weiteren Einzelheiten sind der Tabelle zu entnehmen.

Tabelle 1

| Bsp. | Benzol IV | Carbonsäurechlorid III (immer ...chlorid) | Katalysator Menge | $T_1$ [°C] | $T_2$ [°C] | t [h] | Produkt I (immer ... benzol) | Ausbeute [%] |
|---|---|---|---|---|---|---|---|---|
| 1 | 1,3,5-Trimethyl- | 4-Chlorbenzoyl- 2,2 mol | $Fe_2O_3$ 10 mmol | 100 | 150 | 4 | 1,3-Bis(4-chlorbenzoyl)-2,4,6-trimethyl- | 80 |
| 2 | 1,3,5-Trimethyl- | Benzoyl- 2,2 mol | $Fe_2O_3$ 10 mmol | 100 | 150 | 4 | 1,3-Bisbenzoyl-2,4,6-trimethyl- | 79 |
| 3 | 1,3,5-Trimethyl- | Benzoyl- 6,0 mol | $Fe_2O_3$ 25 mmol | 100 | 180 | 3 | 1,3,5-Trisbenzoyl-2,4,6-trimethyl- | 73 |
| 4 | 1,3,5-Trimethyl- | 4-Nitrobenzoyl 2,2 mol | $Fe_2O_3$ 10 mmol | 120 | 160 | 2 | 1,3-Bis(4-nitrobenzoyl)-2,4,6-trimethyl- | 90 |
| 5 | 1,3,5-Trimethyl- | 2-Methylbenzoyl 2,1 mol | $Fe_2O_3$ 15 mmol | 100 | 150 | 2 | 1,3-Bis(2-methylbenzoyl)2,4,6-trimethyl- | 80 |
| 6 | 1,3-Dimethyl- | Benzoyl- 2,0 mol | $Fe_2O_3$ 25 mmol | 140 | 170 | 2 | Gemisch aus 1,3-Bisbenzoyl-4,5-dimethyl- und 1,3-Bisbenzoyl-2,4-dimethyl- | 60 |
| 7 | 1,2,4,5-Tetramethyl- | Benzoyl- 2,1 mol | $Fe_2O_3$ 5 mmol | 140 | 180 | 2 | 1,4-Bisbenzoyl-2,3,5,6-tetramethyl- | 80 |

| Bsp. | Benzol IV | Carbonsäurechlorid III (immer ...chlorid) | Katalysator Menge | $T_1$ [°C] | $T_2$ [°C] | t [h] | Produkt I (immer ... benzol) | Ausbeute [%] |
|---|---|---|---|---|---|---|---|---|
| 8 | 1,3,5-Triethyl- | Benzoyl- 1,0 mol | $Fe_2O_3$ 5 mmol | 100 | 170 | 3 | 1,3-Bisbenzoyl-2,4,6-triethyl- | 39 |
| 9 | 1,2,3,5-Tetramethyl- | Benzoyl- 2 mol | $Fe_2O_3$ 10 mmol | 150 | 180 | 3 | 1,3-Bisbenzoyl-2,4,5,6-tetramethyl- | 74 |
| 10 | 1,3,5-Trimethyl- | Chloracetyl- 2,3 mol | $Fe_2O_3$ 0,5 mmol | 80 | 100 | 4 | 1,3-Bischloracetyl-2,4,6-trimethyl- (Smp. 129-131°C) | 70 |
| 11 | 1,2,4,5-Tetramethyl- 2 mol | Chloracetyl- 2,5 mol | $Fe_2O_3$ 0,5 mmol | 100 | 100 | 4 | 1,4-Bischloracetyl-2,3,5,6-tetramethyl- (Smp. 196°C) | 55 |
| 12 | 1,2,3,5-Tetramethyl- | Chloracetyl- 2,5 mol | $Fe_2O_3$ 0,5 mmol | 100 | 100 | 4 | 1,3-Bischloracetyl-2,4,5,6-tetramethyl- (Smp. 125-127°C) | 60 |
| 13 | 1,3,5-Trimethyl- | Benzoyl- | $FeSO_4 x 7H_2O$ 10 mmol | 100 | 150 | 4 | 1,3-Bisbenzoyl-2,4,6-trimethyl- | 68 |
| 14 | 1,3,5-Trimethyl- | Benzoyl- | ZnO 20 mmol | 100 | 150 | 4 | 1,3-Bisbenzoyl-2,4,6-trimethyl- | 77 |
| 15 | 1,3,5-Trimethyl- | Benzoyl- | $ZnCl_2$ 20 mmol | 100 | 150 | 4 | 1,3-Bisbenzoyl-2,4,6-trimethyl- | 74 |

| Bsp. | Benzol IV | Carbonsäurechlorid III (immer ...chlorid) | Katalysator Menge | $T_1$ [°C] | $T_2$ [°C] | t [h] | Produkt I (immer ... benzol) | Ausbeute [%] |
|---|---|---|---|---|---|---|---|---|
| 16 | 1,3,5-Trimethyl- | Benzoyl- | $MoO_3$ 20 mmol | 100 | 150 | 4 | 1,3-Bisbenzoyl-2,4,6-trimethyl- | 78 |
| 17 | 1,3,5-Trimethyl- | Benzoyl- | $SnCl_4$ 20 mmol | 100 | 150 | 4 | 1,3-Bisbenzoyl-2,4,6-trimethyl- | 79 |

## Patentansprüche

**1.** Verfahren zur Herstellung von mehrfach acylierten Aromaten der allgemeinen Formel I

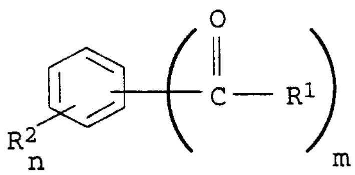

I

in der die Variablen folgende Bedeutung haben:

- $R^1$ Phenyl oder inerte Substituenten tragendes Phenyl, α-Halogen-$C_1$-$C_6$-Alkyl, wobei diese Reste gleich oder verschieden sein können;
- $R^2$ $C_1$-$C_4$-Alkyl;
- m 2 oder 3;
- n 0 bis 4, wobei die Substituenten $R^2$ verschieden sein können, wenn n >1 ist,

mit der Maßgabe, daß $m + n \leq 6$ ist, dadurch gekennzeichnet, daß man einem Acylaromaten der allgemeinen Formel II

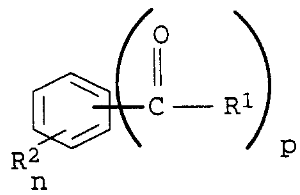

II

in der p für 1 oder 2 steht, mit einer dem gewünschten Acylierungsgrad entsprechenden Menge an einem Carbonsäurehalogenid der allgemeinen Formel III

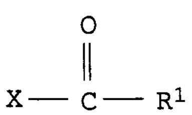

III

in der X für Halogen steht, in Gegenwart von katalytischen Mengen an Fe(II)-, Fe(III)-, Zn(II)-, Mo(VI)-, W(VI)- oder Sn(IV)-Verbindungen umsetzt.

**2.** Verfahren nach Anspruch 1, dadurch gekennzeichnet, daß man Carbonsäurechlorid Cl-CO-$R^1$ umsetzt.

**3.** Verfahren nach Anspruch 1 oder 2, dadurch gekennzeichnet, daß man Tri- oder Tetra-$C_1$-$C_4$-Alkylacylbenzole II umsetzt.

**4.** Verfahren nach den Ansprüchen 1 bis 3, dadurch gekennzeichnet, daß man die Reaktion in Gegenwart von $Fe_2O_3$ vornimmt.

**5.** Verfahren zur Herstellung von mehrfach acylierten Aromaten der allgemeinen Formel I

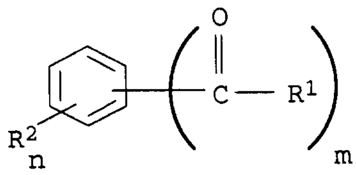

I

in der die Variablen folgende Bedeutung haben:

- $R^1$ Phenyl oder inerte Substituenten tragendes Phenyl, α-Halogen-$C_1$-$C_6$-Alkyl, wobei diese Reste gleich oder verschieden sein können;
- $R^2$ $C_1$-$C_4$-Alkyl;
- m 2 oder 3;
- n 0 bis 4, wobei die Substituenten $R^2$ verschieden sein können, wenn n>1 ist,

mit der Maßgabe, daß $m + n \leq 6$ ist, dadurch gekennzeichnet, daß man einen Aromaten der allgemeinen Formel IV

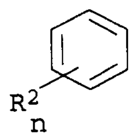

IV

mit einer dem gewünschten Acylierungsgrad entsprechenden Menge an einem Carbonsäurehalogenid der allgemeinen Formel III

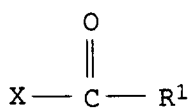

III

in der X für Halogen steht, in Gegenwart von katalytischen Mengen an Fe(II), Fe(III)-, Zn(II)-, Mo(VI)-, W(VI)- oder Sn(IV)-Verbindungen umsetzt.

## Claims

1. A process for preparing polyacylated aromatic compounds of the formula I

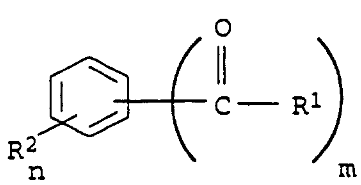

I

where

- $R^1$ is phenyl which is unsubstituted or has inert substituents or is α-halo-$C_1$-$C_6$-alkyl, it being possible for these radicals to be identical or different;
- $R^2$ is $C_1$-$C_4$-alkyl;
- m is 2 or 3;
- n is 0 to 4, it being possible for the $R^2$ substituents to be different when n >1,

with the proviso that $m + n \leq 6$, which comprises reacting an acylaromatic compound of the formula II

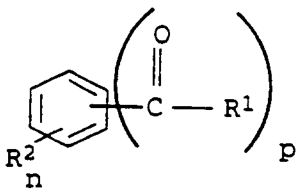

II

where p is 1 or 2, with a carbonyl halide of the formula III

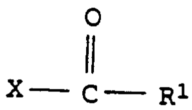

III

where X is halogen, in an amount appropriate for the desired degree of acylation, in the presence of a catalytic amount of Fe(II), Fe(III), Zn(II), Mo(VI), W(VI) or Sn(IV) compounds.

**2.** A process as claimed in claim 1, wherein carbonyl chlorides $Cl\text{-}CO\text{-}R^1$ are reacted.

**3.** A process as claimed in claim 1 or 2, wherein tri- or tetra-$C_1$-$C_4$-alkylacylbenzenes II are reacted.

**4.** A process as claimed in claims 1-3, wherein the reaction is carried out in the presence of $Fe_2O_3$.

**5.** A process for preparing polyacylated aromatic compounds of the formula I

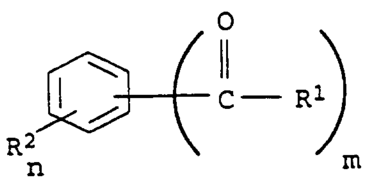

I

where

$R^1$ is phenyl which is unsubstituted or has inert substituents or is $\alpha$-halo-$C_1$-$C_6$-alkyl, it being possible for these radicals to be identical or different;

$R^2$ is $C_1$-$C_4$-alkyl;

m is 2 or 3;

n is 0 to 4, it being possible for the $R^2$ substituents to be different when $n > 1$,

with the proviso that $m + n \leq 6$, which comprises reacting an aromatic compound of the formula IV

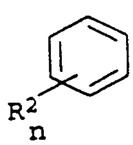

IV

with a carbonyl halide of the formula III

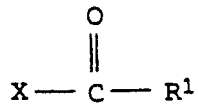

III

where X is halogen, in an amount appropriate for the desired degree of acylation, in the presence of a catalytic amount of Fe(II), Fe(III), Zn(II), Mo(VI), W(VI) or Sn(IV) compounds.

**Revendications**

**1.** Procédé de préparation de composés aromatiques polyacylés, répondant à la formule générale I

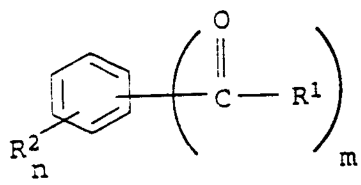

I

dans laquelle les variables ont les significations qui suivent :

$R^1$ représente un radical phényle ou un radical phényle portant des substituants inertes, un radical alkyle en $C_1$ à $C_6$ $\alpha$-halogéné, où ces radicaux peuvent être identiques ou différents,
$R^2$ représente un radical alkyle en $C_1$ à $C_4$,
m est égal à 2 ou à 3,
n a une valeur qui fluctue de 0 à 4, où les substituants $R^2$ peuvent être identiques ou différents lorsque n est supérieur à 1,

avec la condition que $m + n \leq 6$, caractérisé en ce que l'on fait réagir un composé acylaromatique, répondant à la formule générale II

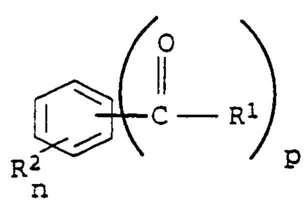

II

dans laquelle p est égal à 1 ou à 2, avec une proportion correspondant au degré d'acylation souhaité d'un halogénure d'acide carboxylique, répondant à la formule générale III

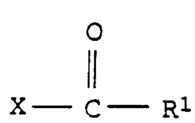

III

dans laquelle X représente un atome d'halogène, en présence de proportions catalytiques de composés de Fe(II)-, Fe(III)-, Zn(II)-, Mo(VI)-, W(VI)- ou Sn(IV).

**2.** Procédé suivant la revendication 1, caractérisé en ce que l'on fait réagir le chlorure d'acide carbonique Cl-CO-$R^1$.

**3.** Procédé suivant la revendication 1 ou 2, caractérisé en ce que l'on fait réagir des tri- ou tétra-alkyl($C_1$-$C_4$)benzènes

II.

4. Procédé suivant l'une quelconque des revendications 1 à 3, caractérisé en ce que l'on entreprend la réaction en présence de $Fe_2O_3$.

5. Procédé de préparation de composés aromatiques polyacylés, répondant à la formule générale I

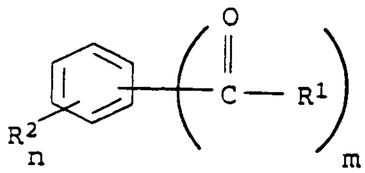

I

dans laquelle les variables ont les significations qui suivent :

- $R^1$ représente un radical phényle ou un radical phényle portant des substituants inertes, un radical alkyle en $C_1$ à $C_6$ $\alpha$-halogéné, où ces radicaux peuvent être identiques ou différents,
- $R^2$ représente un radical alkyle en $C_1$ à $C_4$,
- m est égal à 2 ou à 3,
- n a une valeur qui fluctue de 0 à 4, où les substituants $R^2$ peuvent être identiques ou différents lorsque n est supérieur à 1,

avec la condition que $m + n \leq 6$ , caractérisé en ce que l'on fait réagir un composé aromatique de la formule générale IV

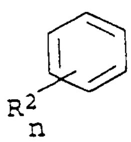

IV

avec une proportion correspondant au degré d'acylation souhaité d'un halogénure d'acide carboxylique, répondant à la formule générale III

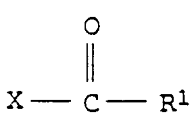

III

dans laquelle X représente un atome d'halogène, en présence de proportions catalytiques de composés de Fe(II)-, Fe(III)-, Zn(II)-, Mo(VI)-, W(VI)- ou Sn(IV).